# EUROPEAN PATENT APPLICATION

(11) **EP 4 575 491 A1**
(43) Date of publication of application: **25.06.2025**
(21) Application number: 23220189.7
(22) Date of filing: 22.12.2023
(51) Int. Cl.: G01N 33/00, G01N 1/40, G01N 33/497

(54) **METHOD FOR DETECTING VOLATILE ORGANIC COMPOUNDS IN FAECAL MATTER ODOUR**

(71) Applicant: Vilnius University, 01513 Vilnius (LT); Valstybinis moksliniu tyrimu institutas Fiziniu ir technologijos mokslu centras, 10257 Vilnius (LT)
(72) Inventor: Burokas, Aurelijus, Vilnius (LT); Bukauskas, Virginijus, Vilnius (LT); Kunevicius, Arnas, Vilnius (LT); Urbonas, Vaidotas, Vilnius (LT); Daugalas, Tomas, Vilnius (LT); Strazdiene, Viktorija, Vilnius (LT)
(74) Representative: AAA Law

(57) **Abstract**

The present invention offers a method for identifying crucial time points marking microbiota changes following diverse interventions, intended for subsequent sequence analyses. This method hinges on measuring the dynamic electrical resistance response triggered by the presence of volatile components extracted from faecal matter. The extraction of these volatile components is achieved through the solid phase microextraction (SPME) technique. Essential parameters of the response signal, including maximal response, response time, and recovery time, are meticulously extracted. The discrimination of microbiota changes is intricately linked to alterations in the odour profile of mice faecal matter. This discernment is accomplished through the application of the principal component analysis (PCA) technique. This method provides a comprehensive and sensitive means to discern and quantify microbiota variations in response to different interventions such as change of feeding.

## Description

### FIELD OF THE INVENTION

The present invention relates to the field of microbiology, bioinformatics and the microbiota research a method for determining most important time points for the changes in the microbiota after various interventions for further sequence analyses.

### BACKGROUND OF THE INVENTION

The field of microbiota research has undergone significant expansion in the past decade, where the gut microbiota became as a therapeutic target in metabolic, immunological and neuropsychiatric disorders [1, 2], even with several patents exploring the relationship between autism spectrum disorder and microbiota (e.g., US2019358274A1, US2023220494A1, WO2023102450A1, US2017360848A1, US2020061148A1). Traditionally, microbiota research relies on sequencing techniques for identifying changes in bacterial communities [3]. However, these methods are expensive, time-consuming, and labor-intensive, limiting the study to a few time points during different interventions.

While metagenomic analysis of nucleic acids from faecal microbiota (e.g., EA034795B1) is informative, the drawbacks of costly and time-consuming procedures persist. Recognizing the diagnostic potential in gaseous emissions of volatile organic compounds (VOCs) from patients, recent studies have demonstrated the ability to detect various diseases from blood, sweat, breath, urine, and faecal samples. Gaseous emissions from urine and faecal samples are particularly valuable for diagnosing gastroenterological and metabolic diseases.

The conventional methods for detecting volatile compounds, such as Gas Chromatography/Mass Spectrometer, Fourier Transform Infrared Spectrometry (FTIR), and Ion Mobility Spectrometry (JMS), are expensive and labor-intensive [4-6]. Conversely, gas sensors offer a cost-effective and rapid alternative to monitor microbiota changes by detecting alterations in metabolites/compounds [7]. Various types of gas sensors, including resistive metal oxide, electrochemical, optical, conducting polymer, quartz crystal microbalance, and pellistor/calorimetric sensors, present promising avenues for this purpose. For instance, an array of chemoresistive sensors has been utilized for screening colorectal cancer/adenomas by measuring VOCs in faecal gases (WO2016063148A1). The proposed method shares similarities with the gas sensor system utilized in WO2016063148A1. Notably, the analysis of the samples is conducted in vitro, making it non-invasive. However, a significant divergence emerges in the methodology applied for diagnostic or change detection output. In patent document WO2016063148A1, a specific algorithm provides either positive or negative diagnosis. In contrast, the proposed method introduces an innovative feature by capturing and considering the continuous change of parameters. Furthermore, the proposed method diverges in its utilization of Solid-Phase Microextraction (SPME) absorbers for the collection of VOCs components, deviating from the conventional approach of relying on airflow through the container. This deviation provides a noteworthy advantage. By selectively choosing the SPME absorber material, specific fractions of VOCs can be intentionally avoided or reduced, while others can be preferentially absorbed. This meticulous control over the composition of collected VOCs enables the selection of key VOCs that are more impactful for the detection of microbiota changes.

The use of array of chemoresistive sensors would make it possible to select exactly the most important time points for the changes in the microbiota after various interventions for further sequence analyses.

### SUMMARY OF THE INVENTION

### Technical Problem

The goal of the present invention is to establish a cost-effective and time-efficient methodology for pointing key time points that denote shifts in microbiota in response to diverse interventions, ranging from alterations in bacterial compositions to dietary changes and beyond.

### Solution to Problem

The present invention is a method for determining the time point when the microbiota of the mammal, for example, mice or humans, begins to change after various interventions such as change of the feeding, and/or physical activity, the faecal microbiota transplantation, medication treatment, pro-, pre-, post-biotics administration, but not limited to. The proposed method is comparably cheap and fast. The method is used for the time points identification of microbiota changes.

This method hinges on measuring the dynamic electrical resistance response triggered by the presence of volatile components extracted from faecal matter. The extraction of these volatile components is achieved through the solid phase microextraction (SPME) technique. Essential parameters of the response signal, including maximal response, response time, and recovery time, are meticulously extracted

### DESCRIPTION OF DRAWINGS

The drawings are provided as a reference to possible embodiments and are not intended to limit the scope of the invention. Neither of the drawings nor the graphs presented herein should be construed as limiting the scope of the invention, but merely as an example of a possible embodiment.
Fig. 1. Typical dynamic resistance response of three individual gas sensors observed after desorption of volatile components from the SPME sorbent.
Fig.2. Typical dynamic resistance response of individual gas sensor observed after desorption of volatile components from the SPME sorbent in various periods after the change of feeding.

### DETAILED DESCRIPTION OF THE INVENTION

The collection of microbiota samples from individual mice involved the careful isolation of each mouse within dedicated chambers. Specifically, when obtaining faecal samples, a deliberate effort was made to exclude urine, a practice crucial for mitigating the impact of strong odours on volatile component measurements. By eliminating the influence of urinary components, the acquired signals are attributed solely to faecal matter, enhancing the precision and reliability of the research outcomes.

A method for mammalian faecal matter odour detection and classification, comprising the steps:
A gas sensor selection from commercially available such as Figaro USA, Inc. TGS2603 and laboratory made resistive tin oxide chemical sensors for the measurement system based on the highest dynamic resistance response signal value change to the vapour of alcohol liquids such as ethyl, propyl, isopropyl.

A procedure for selecting a Solid-Phase Microextraction (SPME) absorber for odour detection sensors, comprising: 1) Collecting volatile organic compounds from the volume containing faecal matter using a commercially available SPME absorbers; 2) Monitoring the resistance response signal parameters of commercially available odour detection sensors such as those produced by Figaro USA, Inc. during the desorption process of the collected volatile organic compounds for a duration of 10 minutes; 3) Selecting an SPME absorber based on the resistance response signal parameters, ensuring optimal desorption efficiency of volatile organic compounds associated with faecal odours.

A calibration procedure of selecting the most different by signal parameter chemical sensors configured to detect volatile organic compounds (VOCs) extracted from the SPME absorber based on the sensor sensitivity and selectivity to ethyl and isopropyl alcohols;

A signal acquisition procedure: volatile organic compounds extraction from the faecal matter and adsorption on SPME absorber, measurements parameter such as sampling rate, desorption conditions (such as absorber heating temperature);

In the process of gathering volatile components from faecal matter odour, a solid phase microextraction (SPME) sampling technique was used. This method entails the utilization of a specialized fibre coated with a solid extracting phase (sorbent) capable of extracting various analytes while avoiding the collection of ambient humidity. To facilitate the manual collection of odours, a custom container was meticulously designed for this purpose. A Solid-Phase Microextraction (SPME) absorber for collecting VOCs of odour was selected by special procedure. VOCs were adsorbed for 10 min on a commercially available SPME absorbers from the volume containing faecal matter and the resistance response of commercially available odour detection sensors produced by Figaro USA, Inc. was monitored during the desorption process. SPME absorber was chosen based on the resistance response signal parameters (signal amplitude, response and decay times). Signal amplitude should be at least 1.5 times higher than the initial one, response time constant should be less than 30 s and response decay time constant should be less than 400 s.

A uniform quantity of individual mice faecal matter was deposited into a small container, coexisting with an SPME sorbent strategically positioned nearby. Extraction time was meticulously tailored, ensuring an optimal 10-minute duration for the comprehensive collection of volatile components constituting the odour. Following this extraction phase, the SPME absorber, now enriched with volatile compounds, was transferred to a chamber housing eight gas sensors reliant on metal oxide films. To enhance the signal-to-noise ratio, these gas sensors were subjected to local heating, reaching temperatures between 350 and 400°C.

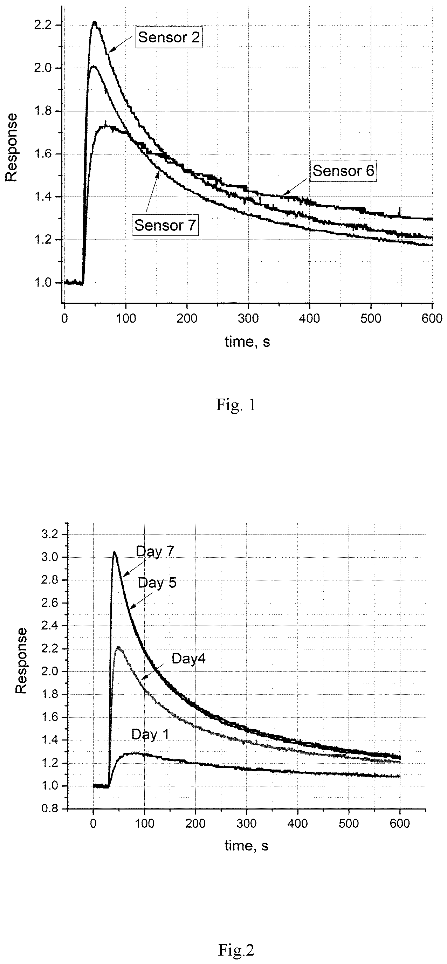

For signal stability, the resistance of each individual gas sensor was diligently measured for up to 1 minute. Subsequently, the end of the SPME absorber, laden with volatile components from the mice faecal sample, underwent sudden controlled heating up to 100°C. This facilitated the efficient desorption of volatile compounds from the sorbent, triggering a pronounced and rapid change in the resistance of each gas sensor. These alterations were promptly recorded at speed of 0.2 seconds per sample and stored in the computer's memory disk for further signal processing. The resistive response to the volatile components was consistently monitored for a minimum of 10 minutes, ensuring a thorough evaluation of the dynamic changes in the gas sensor array. Typical resistance response of 3 individual sensors is shown in Fig.1.

For continuous use, it is imperative to regularly renew the SPME sorbents. This process ensures the sustained efficacy of the sorbents in collecting volatile components, maintaining the reliability and accuracy of the measurements over extended periods. The SPME sorbent was systematically renewed after each measurement through multiple heating cycles at 100°C. This renewal process continued until there was no discernible change in the resistance of any gas sensor after the desorption of volatile components.

To identify changes in the mice microbiota, the resistance response to samples of individual mice faecal matter was measured both before and after modifying the mice's diet. Daily measurements were conducted to pinpoint the specific time at which the response, and consequently the microbiota, underwent changes. This ongoing and systematic comparative analysis of resistance responses serves as a crucial indicator for discerning microbiota variations in response to dietary modifications, among the other factors Fig 2.

Dynamic resistance response signal after the steep change off the atmosphere because of the desorption of the volatile components from the SPME sorbent in the gas sensors' camera was analysed by mathematical methods. Parameters were extracted maximal response, response time, recovery time. These parameters that were collected for individual mice in the individual state of the diet were processed by principal component analysis (PCA) technique. This method enables the discrimination of changes in the odour of individual mice faecal matter by unravelling the intricate relationships between dynamic response parameters and the specific dietary conditions of each mouse.

### References

1. Cai H., Chen X., Burokas A., and Maldonado R. (2023) Editorial: Gut microbiota as a therapeutic target in neuropsychiatric disorders: current status and future directions. Frontiers in Neuroscience 17.
2. Burokas A., Moloney R.D., Dinan T.G., Cryan J.F. (2015) Microbiota Regulation of the Mammalian Gut-Brain Axis. Advances in Applied Microbiology 91: 1-621198291. doi: 10.3389/fnins.2023.1198291.
3. Fricker A.M., Podlesny D., Fricke W.F. (2019) What is new and relevant for sequencing-based microbiome research? A mini-review. Journal of Advanced Research 19:105-112. doi.org/10.1016/j.jare.2019.03.006.
4. Moore J.G., Jessop L.D. and Osborne D.N. (1987) Gas-chromatographic and mass-spectrometric analysis of the odor of human feces. Gastroenterology 93: 1321-1329. doi: 10.1016/0016-5085(87)90262-9.
5. el Manouni el Hassani, S., Soers, R.J., Berkhout, D.J.C., Niemarkt H.J., Weda H., Nijsen T., Benninga M.A., de Boer N.K.H., de Meij T.G.J. and Knobel H.H. (2020) Optimized sample preparation for fecal volatile organic compound analysis by gas chromatography-mass spectrometry. Metabolomics 16, 112. doi:10.1007/s11306-020-01735-6.
6. Ketola R.A., Kiuru J.T., Tarkiainen V., Kokkonen J.T., Räsänen J. and Kotiaho T. (2006) Detection of volatile organic compounds by temperature-programmed desorption combined with mass spectrometry and Fourier transform infrared spectroscopy. Analytica Chimica Acta 562, 245-251. doi: 10.1016/j.aca.2006.01.069.
7. Carmona E.N., Sberveglieri V., Ponzoni A., Galstyan V., Zappa D., Pulvirenti A. and Comini E. (2017) Detection of food and skin pathogen microbiota by means of an electronic nose based on metal oxide chemiresistors. Sensors and Actuators B: Chemical 238, 1227-1230. doi: 10.1016/j.snb.2016.09.086.

## Claims

1. A method for mammalian faecal matter odour detection, comprising the steps:
a) Collecting microbiota samples;
b) Absorbing volatile organic compounds (VOCs) with a Solid-Phase Microextraction (SPME) absorber.
c) Transferring the SPME absorber to a chamber housing eight gas sensors reliant on metal oxide films.
d) Calibrating the sensors;
e) Detecting a signal of the faecal matter odour.

2. The method according to claim 1, wherein VOCs were adsorbed for 10 min on the SPME absorbers from the volume containing faecal matter and the resistance response of signal parameters during the desorption process of the collected VOCs for a duration of 10 minutes were monitored.

3. The method according to claim 1, wherein SPME absorber is selected based on the resistance response signal parameters: signal amplitude at least 1.5 times higher than the initial one, response and decay time constants less than 30 s and 400 s, respectively, ensuring optimal desorption efficiency of volatile organic compounds associated with faecal odours.

4. The method according to claim 1, wherein gas sensor is a chemical sensor with the measurement system based on the highest dynamic resistance response signal value change to the vapour of liquids.

5. The method according to claim 1, wherein the end of the SPME absorber, laden with volatile components from the mice faecal sample, controlled by heating up to 100°C, in order to facilitate the efficient desorption.

6. The method according to claim 1, wherein calibration procedure of the sensor comprising selection of the most different chemical sensor by signal parameter, which is configured to detect VOCs extracted from the SPME absorber.

7. The method according to claim 1, wherein detection of the signal of the faecal matter odour comprises the monitoring the resistive response to the volatile components consistently for a minimum of 10 minutes, ensuring a thorough evaluation of the dynamic changes in the gas sensor array.

8. The method according to claim 1, wherein the signal acquisition procedure comprises volatile organic compounds extraction from the faecal matter and adsorption on SPME absorber.

9. The method according to claim 1, wherein the signal acquisition procedure comprises measurement parameters such as sampling rate, desorption conditions, absorber heating temperature, absorber distance from the faecal sample for volatile organic compounds extraction and adsorption on SPME absorber.

10. The method according to claim 1, wherein the chosen gas sensor array system is specifically employed for identifying alterations in microbiota by detecting changes in the odour signal.

11. The method according to claims 1-10, used for the time points identification of microbiota changes.

12. The method according to claims 1-11, used for identification of microbiota changes in response to dietary and/or physical activity modifications, the faecal microbiota transplantation, medication treatment, pro-, pre-, post-biotics administration.
